(19) Europäisches Patentamt
European Patent Office
Office européen des brevets

(11) Publication number: 0 388 921 A2

(12) # EUROPEAN PATENT APPLICATION

(21) Application number: 90105342.1

(22) Date of filing: 21.03.90

(51) Int. Cl.5: C07D 265/04, C07D 279/04, C07D 261/00, C07D 231/00, C07D 207/00, C07D 263/00, C08K 5/29, C08K 5/35, C08K 5/46, C08K 5/3445, B01F 17/00

(30) Priority: 22.03.89 JP 69458/89
28.04.89 JP 109499/89
11.08.89 JP 206856/89
28.12.89 JP 342197/89
05.01.90 JP 101/90
09.01.90 JP 2311/90
02.02.90 JP 23775/90

(43) Date of publication of application: 26.09.90 Bulletin 90/39

(84) Designated Contracting States: DE FR GB

(71) Applicant: NIPPON ZEON CO., LTD.
6-1, 2-chome, Marunouchi, Chiyoda-ku
Tokyo(JP)

(72) Inventor: Kitahara, Shizuo
1-3-11, Shibatsukahara
Kawaguchi-shi, Saitama-ken(JP)
Inventor: Watanabe, Hiroyuki
669-8 Izumi-cho, Izumi-ku
Yokohama-shi, Kanagawa-ken(JP)
Inventor: Kishimoto, Takuji, Nippon Zeon Isogo-ryo
72-15-33, Isogo-ku
Yokohama-shi, Kanagawa-ken(JP)
Inventor: Toyoshima, Tetuya, Nippon Zeon Isogo-ryo
72-15-33, Isogo-ku
Yokohama-shi, Kanagawa-ken(JP)

(74) Representative: Kraus, Walter, Dr. et al
Patentanwälte Kraus, Weisert & Partner
Thomas-Wimmer-Ring 15
D-8000 München 22(DE)

(54) **Additive for organic solvent or thermoplastic polymer.**

(57) An additive for improving the dispersibility of a particulate substance in an organic medium or a thermoplastic polymer, said additive comprising at least one compound selected from the group consisting of compounds having a 1,3-oxazine structure in the molecule, compounds having a $>C=\overset{\oplus}{N}<$ bond in the molecule and compounds having an oxazolinium ion in the molecule.

EP 0 388 921 A2

## ADDITIVE FOR ORGANIC SOLVENT OR THERMOPLASTIC POLYMER

This invention relates to an additive for improving the dispersibility of a particulate material in an organic solvent or a thermoplastic polymer.

Particulate inorganic substances such as titanium dioxide or zinc oxide are incorporated as pigments in various coating compositions such as paints or printing ink, and particular organic substances are also used as a pigment or a film modifying agent. Inorganic substances such as calcium carbonate or silica are used as reinforcing agents or fillers in thermoplastic polymers and rubbers. Furthermore, chops of monofilaments or organic fine particles such as particulate polymers are incorporated as reinforcing agents or surface-treating agents.

Lubricant oils or fuel oils give off a sludge precursor, a sludge binder, a lacquor, a varnish, a resin component or "soot" during use. When these material flocculate and sediment in the oil, various inconveniences occur. Hence, a detergent-dispersant is used as an essential additive for uniformly and stably dispersing them in the oil without flocculation.

To obtain a dispersion of high performance of a particulate substance in a thermoplastic polymer, it is necessary to disperse fine particles of an inorganic or organic material uniformly in a solid thermoplastic polymer or a solution of the thermoplastic polymer uniformly. Various dispersants or coupling agents have been proposed in order to improve the dispersibility of the inorganic or organic fine particles or to improve adhesion of the fine particles on an interface between the thermoplastic polymer and the inorganic or organic fine particles.

These dispersants or coupling agents include, for example, silane coupling agents, titanium coupling agents, metal salts of fatty acids, zinc salts of high-molecular weigt fatty acids, high-molecular-weight aliphatic hydrophilic fatty acid esters and various surface-active agents. The use of these compounds improves adhesion on an interface between the polymer and the inorganic material, but is not sufficient for the improvement of dispersibility. Furthermore, the type of the particulate substance that can be applied is limited.

It is an object of this invention to provide a novel additive which can be applied to various inorganic and/or organic particulate materials, and improves the dispersibility of inorganic and/or organic particulate materials in thermoplastic polymrers or organic media.

As a result of extensive investigations in an attempt to achieve the above object, the present inventors found that compounds in which a specific heterocyclic structure or a specific atomic grouping is introduced into the molecule (into the molecular chains or the ends of the molecular chains) markedly improve the dispersibility of various inorganic or organic fine particulate substances in organic media and thermoplastic polymers.

The present inventors also discovered that because the above compounds suppress an increase in viscosity of a system to which inorganic and/or organic particulate substances are added, and the addition of these compounds improves the processability, transportability or fillability of an organic medium or a thermoplastic polymer, and a particulate substance surface-treated with the above compounds shows good dispersibility in organic media or thermoplastic polymers.

The present invention is based on these findings.

Thus, according to this invention, there is provided an additive for improving the dispersibility of a particulate substance in an organic medium or a thermoplastic polymer, said additive comprising at least one compound selected from the group consisting of compounds having a 1,3-oxazine in the molecule, compounds having a $>C=\overset{\oplus}{N}<$ bond in the molecule and compounds having an oxazolinium ion in the molecule.

The additive of this invention is a compound which contains at least one compound selected from the group consisting of compounds having a 1,3-oxazine structure in the molecule, compounds having a $>C=\overset{\oplus}{N}<$ in the molecule, and compounds having an oxazolinium ion in the molecule. Specific examples of these compounds are compounds of general formulae (I) to (IIIc) and general formulae (1a) to (7).

The compounds having a 1,3-oxazine structure in the molecule may be heterocyclic compounds having O and N at the 1- and 3-positions in a 6-membered ring, and may include not only compounds having an oxazine ring typically represented by formula (IIIa), but also having a hetero ring in which the double bonds in the 6-membered ring is partly or wholly saturated or the positions of the double bonds are different such as a 4H, 5H-1,3-oxazine ring of, for example, formula (IIc). Specific examples of the compounds having a $>C=\overset{\oplus}{N}<$ bond in the molecule are (1) compounds having a 1,3-oxazine structure in which N in the heterocycle is in the form of a quaternary ammonium salt [formulae (Ia) and (Ib)]; (3) compounds having a 1,3-thiazine ring or a 5H,6H-1,3-thiazine ring in which N in the thiazine ring is in the form of a quaternary

ammonium salt [formulae (2a) and (2b)]; (3) compounds having an isoxazole ring or an isothiazole ring or a 4H,5H-isoxazole ring (or isothiazole ring) in which N in the isoxazole ring (or isothiazole ring) is in the form of a quaternary ammoniuim salt [formulae (3a) and (3b)]; (5) compounds having a 1,2-diazole ring or a 4H, 5H-1,2-diazole ring, in which N in the diazole ring is in the form of a quaternary ammonium salt [formulae (4a) and (4b)]; and (6) compounds having a 2H-pyrrole ring or a 2H,3H,4H-pyrrole ring, in which N in the pyrrole ring is in the form of a quaternary ammonium salt [formulae (5a) and (5b)]. Other examples include reaction products obtained by the reaction of polymers (or oligomers) having bonded thereto alkali and/or alkaline earth metals, with reactants which on reaction with the above polymers produce a $>C=\overset{\oplus}{N}<$ bond, for example, 4,4′-bis(diethylamino)benzophenone [for example, formula (6)]. Examples of the compounds having an oxazolinium ion in the molecule are polymers [for example, formula (7)] obtained by cationically polymerizing a 2-oxazoline, and coagulating the resulting polymer with a solvent having no nucleophilic reactivity.

These compounds may include, for example, compounds obtained by introducing atomic groupings of the following general formula in the molecule (into the molecular chains or into the ends of the molecular chains) by using compounds having a carbon-carbon unsaturated bond in the molecular chains (main chains or side chains), for example, polymers having an unsaturated bond in the molecular chains such as alpha-olef ins having a long-chain alkyl groups, low-molecular-weight polyethylene, oligomers of alpha-olefins or poly(styrene methacrylate), or polymers to which alkali and/or alkaline earth metal salts bonded thereto by the chemical reactions to be described below, or by cationic polymerization of 2-oxazolines.

[Compounds having a 1,3-oxazine structure]

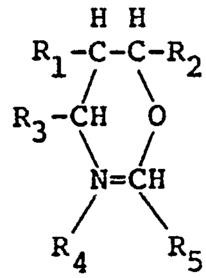

(I)

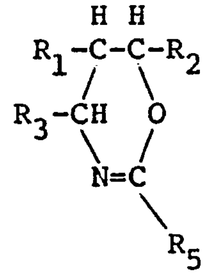

(IIa)

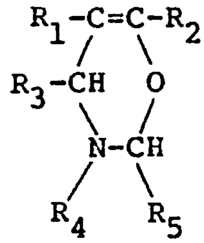

(IIb)

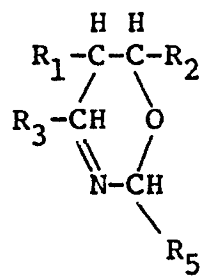

(IIc)

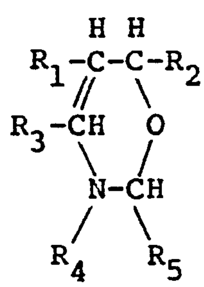

(IId)

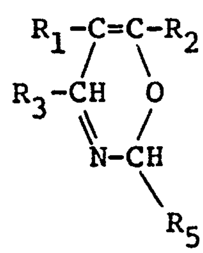

(IIIa)

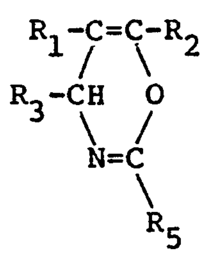

(IIIb)

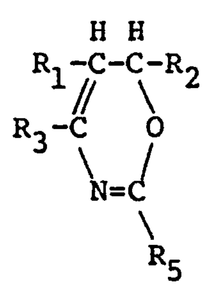

(IIIc)

[Compounds having a >C=$\overset{\oplus}{N}$< bond]

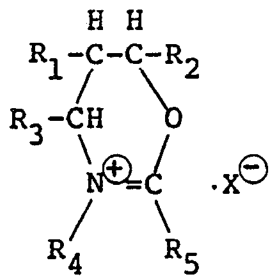

(1a)

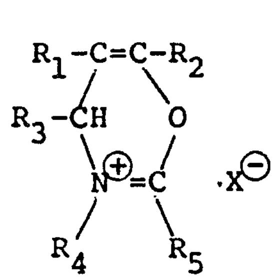

(1b)

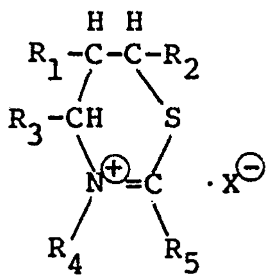

(2a)

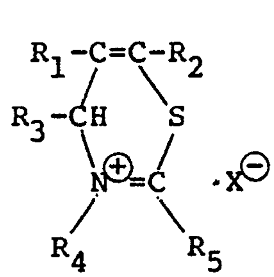

(2b)

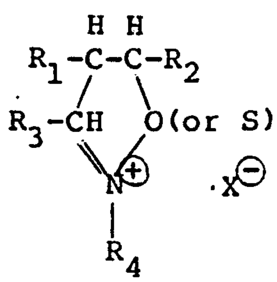

(3a)

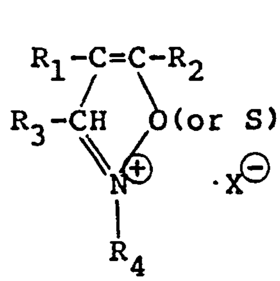

(3b)

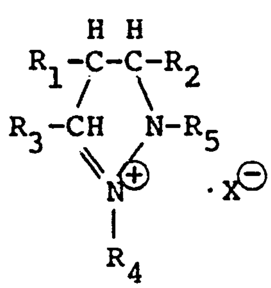

(4a)

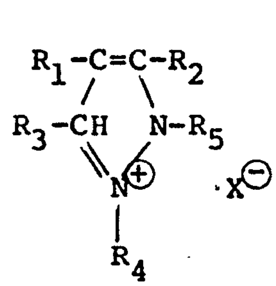

(4b)

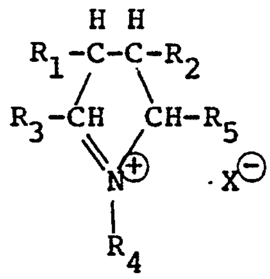

(5a)

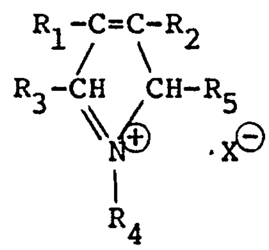

(5b)

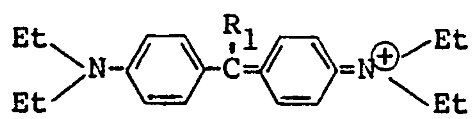

(6)

[Compounds having an oxazolinium ion]

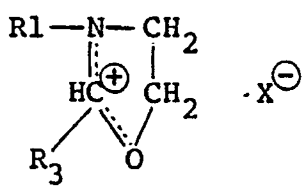

(7)

In the above formulae, $R_1$ represents the main chain of each compound; $R_2$ represents the main chain of the compound; a hydrogen atom or a hydrocarbon group having not more than 6 carbon atoms; $R_3$ to $R_5$ may be identical or different and each represents a hydrocarbon group at least part of which is substituted by a substituent such as a halogen atom, a nitro group, an epoxy group, a carboxyl group or a hydroxyether group; and $X^{\ominus}$ represents an anion.

Some of the methods of producing the additive of this invention are shown below.

(I) The compounds containing a heterocycle having a 1,3-oxazine structure or a $>C=\overset{\oplus}{N}<$ bond may be produced by starting from compounds having a carbon-carbon unsaturated bond such as a carbon-carbon double bond or a triple bond in the molecular chains (at the ends or in the molecular chains).

Examples of the starting compounds having an unsaturated bond at the ends of the molecular chain include alpha-olef ins having a long-chain alkyl group such as 1-decene, 1-dodecene, 1-tetradecene, 1-hexadecene, 1-eicosene and 1-tridecene; low-molecular weight polymers and oligomers such as low-molecular-weight polyethylene, liquid or low-molecular-weight polypropylene and oligomers of alpha-olefins; and macromers such as polybutene, polyisobutylene, polyethylene glycol dimethacrylate, polyethylene glycol diallylate, polypropylene glycol dimethacrylate, polystyrene methacrylate and polystyrene allylate. Examples of the starting compound having an unsaturated bond in the molecular chain are organic compounds having a -C=C- bond, such as 1,9-decadiene, 2,3-dimethylbutene, 2,5-hexadiene, 7-tetradecene and 2,4,4-trimethyl-2-pentene; homopolymers or copolymers such as butadiene, isoprene, piperylene, dicyclopentadiene and ethylidenenorbornene; and copolymers of con jugated dienes and vinyl monomers. Specific examples of these compounds are polybutadiene, polyisoprene, a styrene-butadiene random copolymer, styrene-butadiene block copolymers (A-B type, A-B-A type; A represents a polystyrene block, and B represents a polybutadiene block), a styrene/isoprene random copolymer, styrene/isoprene

block copolymers (A-B type and A-B-A type; A represents a polystyrene block and B represents a polyisoprene block), acrylonitrile/butadiene copolymer, butadiene/propylene block copolymer and ethylene/propylene/diene-monomer copolymers, and partially hydrogenated products (with an iodine number of 5 or more) of the above (co)polymers.

Examples of the compounds having a -C≡C- bond include organic compounds, such as 1-decyne, 3,3-di-methyl-1-butyne, hepyne, hexyne, 1,8-nonadiyne and octyne. Compounds which can be used as starting materials in this invention are compounds having a carbon-carbon unsaturated bond in the ends of the molecular chains or in the molecular chains and being soluble, or compatible with organic media or thermoplastic polymers. There is no particular restriction on the molecular weight of these compounds. They may include low-molecular-weight organic compounds, polymers ranging from oligomers to high-molecular-weight-polymers having a molecular weight of about several hundred thousand). The optimum molecular weight of these polymers may be selected according to the medium used. Typical methods of production are shown below.

(1) A method comprising reacting a compound having a carbon-carbon unsaturated bond with an organic compound of formula (a)

$Y_1$-CH = N-$Y_2$ (a)

in which $Y_1$ and $Y_2$ represent an organic atomic grouping,

and an organic acid halide in the presence of a Lewis acid.

The organic compound of formula (a) denotes a compound in which specifically $Y_1$ and $Y_2$ represent an aliphatic, alicyclic or aromatic residue which may optionally have an alkoxy, cyano, carboxyl or dialkylamino group. Specific examples of the organic compound (a) include Schiff base compounds such as benzylidene methylamine, benzylidene butylamine, benzylidene aniline, benzylidene cyclohexylamine, propylidene aniline, ethoxybenzylidene butylamine, 4-carbomethoxybenzylidene butylaniline, benzylidene-4-cyanoaniline and dimethylaminobenzylidene butylaniline.

Specific examples of the organic acid halide are acetyl chloride, acetyl bromide, benzoyl chloride, acryloyl chloride, carbomethoxybenzoyl chloride, cinnamoyl chloride and methacryroyl chloride.

Examples of the Lewis acid are $BF_3$, $BF_3O(C_2H_5)_2$, $AlCl_3$, $TiCl_4$, $SnCl_4$, $SbCl_5$, and $AgBF_4$. There is no particular restriction on the reaction conditions. Usually, the reaction is carried out at a temperature of 20 to 80 °C for about 1 to 2 hours in an inert solvent such as benzene, toluene or cyclohexane. Usually, the amounts of the organic compound (a) and the organic acid halide are about 1 to 1.5 moles per mole of the unsaturated compound. The amount of the Lewis acid used is about 0.1 to 1 mole per mole of the organic acid halide. If the unsaturated compound is a polymer, the amounts of the organic compound (a) and the organic acid halide are about 0.1 to 30 parts by weight per 100 parts by weight of the polymer.

This method gives compounds having a heterocyclic structure containing a $>C=\overset{\oplus}{N}<$ bond.

(2) A method which comprises reacting the above compound having a carbon-carbon unsaturated bond in the molecular chain with an N-hydroxymethylamide compound (N-methylol compound) in the presence of a Friedel-Crafts catalyst, and as required, further reacting the product with an alkyl halide, methyl p-toluenesulfonate or dimethyl sulfate.

The N-hydroxymethylamide compound is a reaction product between an amide compound and an aldehyde compound. Examples of the aldehyde compound are aliphatic and aromatic aldehydes such as formaldehyde, butyraldehyde, valeraldehyde and benzaldehyde. Examples of the amide compound are acetamide, benzamide, methoxybenzamide, nitrobenzamide, N-methylbenzamide, butyramide, phthalamide and glutaramide. Copolymers containing an N-methylolacryalmide monomer as one component may also be used as the N-hydroxymethylamide compound.

Examples of the alkyl halide are mainly benzyl bromide, benzyl chloride, bromohexane, bromopropane, 2-chloroethyl ether, chloromethyl ether and chloropentane.

Generally known Friedel-Crafts catalysts may be used in this reaction. Typical examples are halogen compounds of metals or semimetals, for example, halogen-compounds, organic halides or complexes of elements such as B, Al, Si, P, Ti, V, Fe, Zn, Mo, Sn, Sb, Te and W, or of oxygen-element compounds such as PO, $SO_2$ and VO. More specific examples include $BF_3$, $BF_3O(C_2H_5)_2$, $BCl_3$, $AlCl_3$, $TiCl_4$, $SnCl_4$, $FeCl_3$, $WCl_5$, $POCl_3$, and $(C_2H_5)_3Al$.

There is no particular restriction on the reaction conditions [the details of this reaction are shown, for example, in C. Giordano et al., SYNTHESIS, 92 (1971)].

(3) A method which comprises reacting the compound having a carbon-carbon unsaturated bond in the molecular chain with nitrile oxide, nitrile imine or nitrile ylide (known as a 1,3-dipole addition reaction), thereby perfoming N-alkylation.

For details of the 1,3-dipole addition reaction, see Huisgen, Angew. Chem., 75, 604 (1863). The reaction of introducing an isooxazoline ring by nitrile oxide is described in Tada and Numata et al., Journal of the

Society of Rubber Industry, Japan 43, 996 (1970), and the reaction of introducing a pyrazoline ring by nitrile imine is disclosed in Caraculacu et al. Polym. Lett. 6, 451 (1968).

(4) A method which comprises reacting the compound having a carbon-carbon unsaturated bond with a halo-hydroximino compound in the presence of a dehydrochlorinating agent such as anhydrous sodium carbonate, and as required, further reacting the produce with an alkyl halide or dimethyl sulfate to perform N-methylation.

The halohydroximino compound can be obtained by reacting an alpha-haloaceto compound described in T. L. Gilchrist et al., J. C. S. Chem. Commun., 1090 (1979) with hydroxylamine hydrochloride , or by reacting a vinyl compound such as acrolein, an acrylic acid ester or alpha-methylstyrene described in K. A. Ogloblin et al., J. Org. Chem., U. S. S. R. 1, 1370 (1965) with nitrosil chloride.

The synthesis of a compound having an oxazine structure by the reaction of an olef in with a halohydroximino compound shown in a synthesis example hereinbelow was in accordance with the method of T. L. Gilchrist et al., J. Chem. Soc. Perkin Trans. I 1275 (1983).

The synthesis of compounds having an oxazine structure is also described in detail in H. E. Zaugg et al. Synthesis, 85 (1984), Synthesis 182 (1984) and Synthesis 182 (1984).

The compounds having a heterocycle which are obtained by the above methods may be partly substituted by a substituent such as a halogen atom, a nitro group, an epoxy group, a carboxyl group or a hydroxy ether group.

(II) Other methods of producing compounds having a $>C=\overset{\oplus}{N}<$ bond in the molecule include a method which comprises reacting a living anion polymer having an alkaline metal and/or an alkaline earth metal at the ends obtained by polymerizing a monomer with a catalyst based on the alkali metal and/or alkaline earth metal (the so-called anion polymerization catalyst) with the organic compound described hereinbelow, and then hydrolyzing the reaction product, and a method which comprises reacting a polymer obtained by adding the above metals to an unsaturated polymer having a double bond in the polymer chain or in a side chain by after-reaction, with the organic compound described hereinafter and hydrolyzing the reaction product (U. S. Patents Nos. 4,550,142 and 4,647,625).

The polymerization catalyst used heretofore in anionic polymerization can be used as the above polymerization catalysts based on the above metals, and there is no particular restriction. Typical examples of the alkali metal-base catalysts include organic lithium compounds having 2 to 20 carbon atoms such as n-butyllithium and sec-butyllithium. Examples of the alkaline earth metal-base catalysts are catalyst systems containing barium, strontium and calcium compounds disclosed in U. S. Patents Nos. 3946385, 3992561, 4079176, 4092268, 4112210, 4129705, 4260519 and 4297240. The polymerization reaction and the reaction of adding an alkali metal and/or alkaline earth metal are carried out in hydrocarbon solvents heretofore used in anionic polymerizations or in solvents which do not destroy the above metal-base catalysts, such as tetrahydrofuran, tetra hydropyran and dioxane. Examples of monomers that can be used in polymerization are the monomers which constitute the polymers or copolymers described hereinbelow. Examples of polymers used to add the above metals by after-reaction are various diene (co)polymers and partial hydrogenation products of these, and ethylene/propylene/diene monomer copolymers.

A preferred group of organic compounds which are used in reaction with the aforesaid polymers having the metals bonded thereto are shown below.

Compounds having a $-\underset{\underset{X}{\|}}{C}-N$ bond in the molecule (in which X is an oxygen or sulfur atom) such as N-substituted lactams and the corresponding thiolactams such as N-methyl-beta-propiolactam, N-t-butyl-beta-propiolactam, N-phenyl-beta-propiolactam, N-methoxyphenyl-beta-propiolactam, N-naphthyl-beta-propiolactam, N-methyl-2pyrrolidone, N-t-butyl-2-pyrrolidone, N-phenyl-3-pyrrolidone, N-methoxyphenyl-2-pyrrolidone, N-vinyl-2-pyrrolidone, N-benzyl-2-pyrrolidone, N-naphthyl-2-pyrrolidone, N-methyl-5-methyl-2-pyrrolidone, N-t-butyl-5-methyl-2-pyrrolidone, N-phenyl-5-methyl-2-pyrrolidone, N-methyl-3,3′-dimethyl-2-pyrrolidone, N-t-butyl-3,3′-di-methyl-2-pyrrolidone, N-phenyl-3,3′-dimethyl-2-pyrrolidone, N-methyl-2-piperidone, N-t-butyl-2-piperidone, N-phenyl-2-piperidone, N-methoxyphenyl-2-piperidone, N-vinyl-2-piperidone, N-benzyl-2-piperidone, N-naphthayl-2-piperidone, N-phenyl-3,3′-dimethyl-2-pyrrolidone, N-methyl-epsilon-caprolactam, N-phenyl-epsilon-caprolactam, N-methoxyphenyl-epsilon-caprolactam, N-vinyl-epsilon-caprolactam, N-benzyl-epsilon-caprolactam, N-naphthyl-epsilon-caprolactam, N-methyl-omega-laurolactam, N-phenyl-omega-laurolactam, N-t-butyl-omega-laurolactam, N-vinyl-omega-laurolactam and N-benzyl-omega-laurolactam, and N-benzyl-omega-laurolactam; N-substituted cyclic ureas and the corresponding N-sub stituted thiocyclic ureas, such as 1,3-dimethyl-2-imidazolidinone, 1,3-diethyl-2-imidazolidinone, 1-methyl-3-ethyl-2-imidazolidinone, 1,3-dimethylethyleneurea, 1,3-diphenylethyleneurea, 1,3-di-t-butylethyleneurea and 1,3-divinylethyleneurea, and N-substituted aminoketones and the corresponding N-substituted aminothioketones, such as 4-dimethylaminobenzophenone, 4-diethylaminobenzophenone, 4-di-t-butylaminobenzophenone, 4-diphenylbenzophenone, 4,4'-bis(dimethylamino)benzophenone, 4,4'-bis-(diethylamino)benzophenone, 4,4'-bis(di-t-butylamino)-benzophenone, 4,4'-bis(diphenylamino)benzophenone, 4,4'-bis(divinylamino)benzophenone, 4-dimethylaminoacetophenone, 4-diethylaminoacetophenone, 1,3-bis(diphenylamino)2-propanone, and 1,7-bis-(methylethylamino)-4-heptanone; and N-substituted aminoaldehydes and the corresponding N-substituted aminothioaldehydes, such as 4-dimethylaminobenzoaldehyde, 4-diphenylaminobenzoaldehyde and 4-divinylaminobenzaldehyde. The amount of these compounds to be used is preferably 0.005 to 10 moles per mole of the anion living polymer and the alkali metal and/or alkaline earth metal-base catalyst used at the time of bonding these metals by after-reaction. If it is less than 0.05 mole, the function of the resulting polymer as a dispersant is insufficient. If it exceeds 10 moles, the resulting polymer becomes difficult to dissolve in the medium because of a side-reaction. The particularly preferred amount is 0.2 to 2 moles. The reaction usually proceeds for a period of several seconds to several hours at room temperature to 100 °C. After the reaction, the desired polymer having the above functional group bonded thereto is recovered from the reaction solution by using a proton-donating solvent (such as water or an alcohol) to coagulate the resulting polymer, usually by steam stripping.

The above reaction is shown specifically by using 4,4'-bis(diethylamino)-benzophenone. In the for mulae, P-Li represents an Li-terminated polymers and P represents a polymer chain.

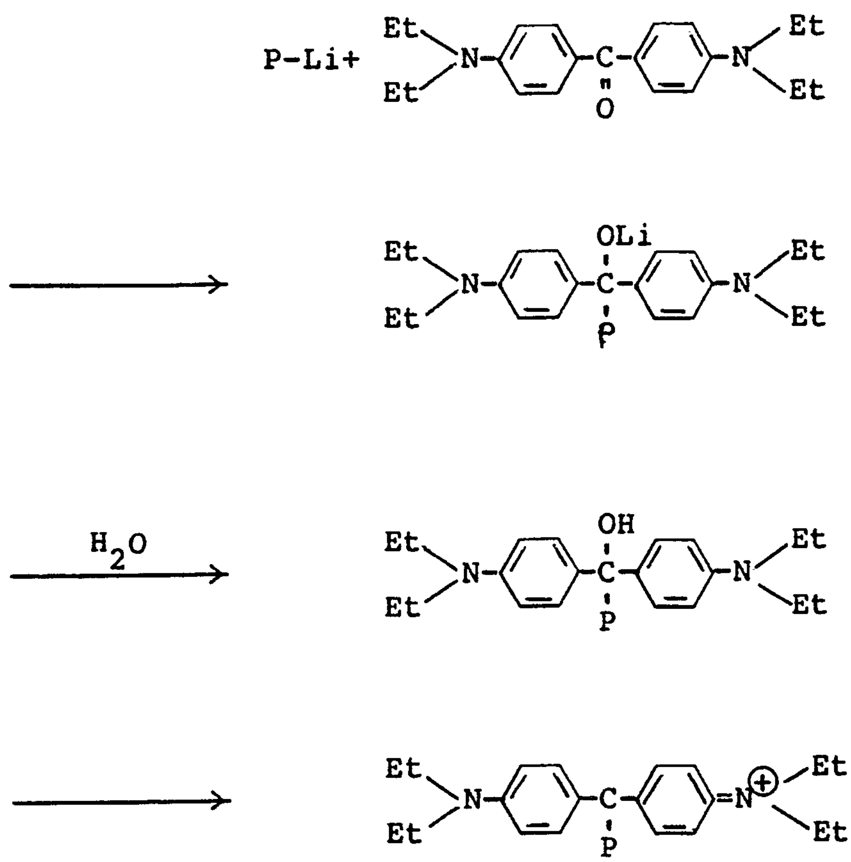


Especially preferred as the additive of this invention are conjugated diene polymers in which the above functional groups are bonded to one or both ends of the polymer chain, Specific examples include homopolymers and copolymers of conjugated dienes polymerizable with the above polymerization catalysts, such as buta diene, isoprene, 2,3-dimethylbutadiene, pentadiene and chloroprene; and copolymers of at least one conjugated diene with at least one monomer copolymerizable with it (for example, aromatic vinyl compounds such as styrene, vinyltoluene and alpha-methylstyrene, unsaturated nitrile compounds such as acrylonitrile and methacrylonitrile, unsaturated carboxylic acids such as acrylic acid, methacrylic acid, maleic acid and maleic anhydride, and unsaturated carboxylic acid esters such as methyl acrylate, ethyl acrylate, butyl acrylate and methoxyethyl acrylate). But the additive of this invention should not be limited to these examples, and suitable polymers (which dissolve in the medium or mutually dissolve with

the medium) as the additives may be selected depending upon the type of the medium in which the inorganic or organic fine particles are to be dispersed. Likewise, there is no particular restriction on the molecular weight of these polymers, and they include compounds ranging from oligomers to higher-molecular-weight polymers (with a weight average molecular weight of about several hundred thousand). The optimum range of molecular weights may be selected depending upon the type of the media.

(III) The compounds having an oxazolinium ion are polymers (including oligomers) having an oxazolinium ion in the molecular chain or at the ends of the molecular chain. These polymers can be produced by polymerizing 2-oxazolines of the formula

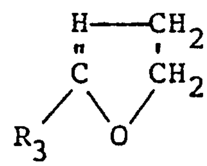

wherein $R_3$ is as defined above, with a cationic polymerization catalyst, and using a solvent having no nucleophilic reactivity as a polymerization stopper and a coagulating agent. If a solvent having nucleophilic reactivity, such as water, ammonia or a primary amine, is used as the polymerization stopper and the coagulating agent, an oxazolinium ion is not formed, but a hydroxyl group or a secondary amino group is formed. Thus, a polymer meeting the object of the invention cannot be obtained.

Typical examples of the 2-oxazolines are 2-oxazoline, 2-methyl-oxazoline, 2-ethyl-oxazoline and 2-substituted oxazolines having substituents other than methyl and ethyl may also be used if the substituents do not inhibit the polymerizatrion reaction.

Ordinary cationic polymerization catalysts, such as p-toluenesufonate esters, benzenesulfonate esters, methanesufonate esters, trifluoromethanesulfonate esters, alkyl iodides, alkyl bromides, alkyl chlorides, benzyl bromides and benzyl chlorides may be used in this polymerization. There may also be used polymeric catalysts which dissolve in, or mutually dissolve with, the medium used in this invention. Examples of the latter include polymers having a proton-donating group such as a halogen atom, a sulfoxyl group or a phosphoxyl group, such as polyvinyl chloride, vinyl chloride/vinyl acetate copolymer, polyepichlorohydrin, epichlorohydrin/ethylene oxide copolymer, and a sulfoxyl group-terminated polymer obtained by reacting a hydroxyl-terminated polymer with p-toluenesulfonyl chloride.

Acetonitrile, benzonitrile, nitromethane, toluene and benzene may be among solvents that can be used in this polymerization.

The polymerization is carried out usually at -20 to 150 °C, preferably 0 to 120 °C.

By using a solvent having no nucleophilic reactivity, such as methanol, ethanol, diethyl ether, n-hexane and petroleum ether, as a polymerization stopper and a coagulating agent after the polymerization, a polymer having the desired oxazolinium ion is obtained. One example of the polymerization is shown as follows:-

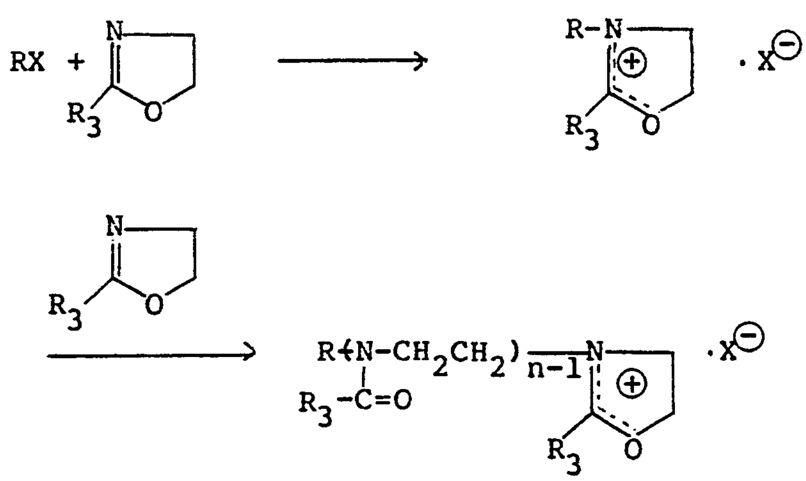

In these formulae, R represents a monovalent organic group, $R_3$ is as defined above, n represents a positive integer, and X represents an electrophilic group.

Details of this polymerization are disclosed, for example, in S. Kobayashi et al., Encyclo. Polymer Sci. & Eng., Vol. 4 (2nd Ed.), 525 (1986).

The additive of this invention can be produced by the methods described above. The chemical structure of the backbone of compounds to which the aforesaid atomic groupings are bonded should be such that the compounds dissolve in, or mutually dissolve with, an organic medium or a thermoplastic polymer for which the additives of the invention are used. It may be selected according to the type of the medium. The molecular weight of the additive, which may vary depending upon the type of the medium, is usually 100 to 300,000 in weight average molecular weight (Mw).

At least one atomic grouping described hereinabove may be present in the molecule.

The particulate material for which the additives of the invention are used is an inorganic or organic substance which are in the form of a powder, flake or fiber. There is no particular limitation on the size of the particulate substance, and may usually be not more than 0.1 mm, preferably 0.01 to 50 micrometers.

Specific examples of the inorganic particulate substance include inorganic reinforcing materials such as metals, clay, carbon black, calcium carbonate, barium sulfate, silica, mica, glass and asbvestos; metal compounds such as compounds of zinc, magnesium, lead and aluminum as reactive inorganic materials; inorganic pigments such as titanium dioxide, iron oxide and zinc chromate; inorganic fillers such as zinc oxide, magnesium oxide, antimony oxide, barium ferrite, strontium ferrite, berylium oxide, pumice, aluminum hydroxide, magnesium hydroxide, basic magnesium carbonate, dolomite, calcium sulfate, ammonium sulfate, calcium sulfite, talc, mica, glass balloon, glass beads, glass fibers, calcium silicate, montmorillonite, bentonite, grahite, carbon fibers, molybdenum sulfide, boron fibers, silicon carbide fibers, zinc borate, barium meta-borate, and zirconium titanate; sludges precipitated from heavy oils during storage; carbon coming from the combustion chamber of an internal combustion engine into a lubricant oil within a crankcase; and sludges formed during use of a lubricant oil.

Specific examples of the organic particulate substance include low-molecular-weight organic compounds such as anthracene, pyrene and ferrocene; waxes precipitated at low temperatures lubricant in oils or fuel oils, as residues of distillation at atmospheric or reduced pressure of petroleum or naphaha and asphalthene; polycondensation products of imperfectly burnt products of fuel oils which come into a lubricant oil in a rankcase from the combustion chamber of an internal combustion engine; organic pigments such as azo pigments, mordant dye-type lakes, phthalocyanine pigments and organic fluorescent pigments; fine particles of polyethylene, polystyrene, benzoguanamine resin, methacrylic acid resin, silicone resins, polyamide resins, polyester resins, polyphenylene oxide, polyphenylene sulfide, and phenolic thermosetting resins; and chops of fibers such as aramid fibers, polyamide fibers and polyester fibers. Examples of the organic media in this invention include organic solvents such as benzene, toluene, hexane, cyclohexane, methyl ethyl ketone, acetone, methyl acetate and alcohols; and base oils for lubricant oils [e.g., gasoline engine oils, Disel engine oils, gear oils, turbine oils, acuating oils, rubber-incorporated oils (such as aromatic or naphthetic process oils and waxes], such as mineral oil type base oils, and alpha-olefin type synthetic base oils or ester-type synthetic oils; and intermediate distillation petroleum fuel oils having a boiling point of 40 to 500 °C (such as gasoline, light oils, kerosene, diesel fuel oils, jet fuel oils and A heavy oil). These examples are not limitative.

Examples of the thermoplastic polymers for which the additive of the invention are used include thermoplastic resins such as vinyl chloride resins, ABS resin, AS resin, polyethylene, polypropylene, polystyrene, high impact polystyrene, polyethylene terephthalate, polycarbonates, polyamides and polyphenylene ether; and elastomers and parital hydrogenation products of these, such as natural rubbers, polyisoprene, polybutadiene, styrene/butadiene copolymer, acrylonitrile/butadiene copolymer, ethylene/propylene/ethylidenenorbornene copolymer, ethylene/propylene/diene copolymers, styrene/butadiene block copolymer, styrene/butadiene/styrene block copolymer, styrene/isoprene block copolymer and styrene/isoprene/styrene block copolymer. These examples are not limitative. The additives of the invention may be used singly or in combination. The amount of the additive may vary depending upon the type of the medium, or the content of the above-specified atomic the above-specified atomic grouping in the additive. Usuallly, it is at least 0.05 part by weight, preferably 0.1 to 10 parts by weight, especially preferably 0.5 to 20 parts by weight.

When the medium is a lubricant oil or a fuel oil, the additive is used normally in an amount of 0.01 to 20 % by weight based on the oil.

For example, there are the following methods of using the additive.

(1) The additive is added in advance to an organic medium other than lubricant oils and fuel oils, or a thermoplastic polymer, and then the particulate substance is added.

(2) The additive are added together with the particulate substance to the organic medium or the thermoplastic polymer.

(3) The particulate substance is added to the organic medium or the thermoplastic polymer, and then the additive is added.

(4) The particulate substance is surface-treated in advance with the additive, and then added to the organic medium or the thermoplastic polymer.

When the particulate substance surface-treated in advance with the additive is to be used, the amount of the additive for the surface treatment is usually at least 1 % by weight, preferably at least 3 % by weight, based on the particulate substance although it may vary depending upon the molecular weight of the additive, and the content of the above-specified atomic grouping.

The method of surface-treating the particulate substance with the additive is not particularly limited. For example, the additive is dissolved in an inert organic solvent, and then the particulate substance is immersed in the resulting solution. Alternatively, a solution of the additive or a suspension of the additive in an insoluble medium such as water is coated with the substance. A dry blending method may also be applied.

The thermoplastic polymer should be mixed sufficiently with the particulate substance in order to produce a uniform dispersion of the particulate substance in the thermoplastic polymer by using the additive of the invention and the surface-treated particulate substance. The preferred mixing conditions depend upon the type of the polymer.

It is possible to surface-treat the particulate substance with the additive by using a kneader of an ordinary type, such as a Henschel mixer, a Banbury mixer, or a double concentric screw, and then mix the surface-treated substance with the thermoplastic polymer.

Generally, a polymer is processed, for example, mixed under a high shear at a temperature far exceeding the second order transition temperature of the polymer, desirably at a temperature at which the polymer has a low melt viscosity. Accordingly, the processing of a thermoplastic polymer containing the additive of the invention is also carried out at the above temperature. Examples of the mixing apparatus are the aforesaid mixing machines, a two roll mill, and a Waring blendor normallly used for polymer processing.

The additive of this invention used to disperse a particulate substance uniformly in the thermoplastic polymer should be a polymer having a backbone, or a monomer composition, compatible with the polymer. If its molecular weight is too low, the additive may bleed out onto the surface of the resulting shaped article. Preferably, the additive has a weight average molecular weight of 1,000 to 300,000. When the particulate substance is dispersed in an organic solvent for a paint or a magnetic fluid, the additive of this invention must be soluble in the organic solvent used, and the additive is selected according to the organic solvent. In this case, the additive has a preferable molecular weight of usually 100 to 10,000 in weight average molecular weight although it depends upon the amount of the additive used.

When the additive of this invention is used in a lubricant oil or a fuel oil, the additive should dissolve in these oils as in the above case. It should be a polymer having a backbone soluble in these oils.

The preferable weight average molecular weight of the additive is usually 1,000 to 100,000 although it depends also upon its amount when it is for fuel oils. For lubricant oils, its weight average molecular weight is usually preferably in the range of 1,000 to 100,000. When the additive of this invention is added to a lubricant oil, it also exhibits the function of a viscosity index improver, a low temperature flow modifying agent or an antioxidant. When the additive is to be added to a lubricant oil or a fuel oil, it is the general practice to first dissolve it in a diluent solvent which dissolves the additive, and then add the diluted additive to the oil and thereafter remove the solvent.

The additive of this invention facilitates the uniform dispersion of the particulate substances in organic media or in thermoplastic polymers, and suppresses the rise of the viscosity of a system to which the particulate substance has been added. Accordingly, it offers a great advantage of markedly increasing the amount of the particulate substacnce.

The additive of this invention is multifunctional in that it imparts better low temperature flowability and detergent-dispersibility as compared with the prior art, and to lubricant oils, it gives detergent-dispersibility and increases the viscosity index.

The additives of this invention are useful, for example, as dispersing agents for a variety of materials such as magnetic materials for bonded magnets, magnetic recording materials (for toners, disks or tapes) or magnetic fluids, electrically condutive materials for paints, molded articles for EMI, fire retarding materials for cable coverings or building materials, pigments, fillers, reinforcing agents, catalysts, reflection preventing agents, dyes or FRTP, and also as detergent-dispersing agents for lubricant oils or fuel oils.

The additives of this invention may also be used in the fields of paints, adhesives and cosmetics.

The following Production Examples for the additives of the invention, Examples and Comparative

Examples illustrate the invention further. It should be understood that the invention are not to be limited to these examples. In these examples, all parts and percentages are by weight unless otherwise specified.

The presence of $>C=\overset{\oplus}{N}<$ was derteremined by an ultraviolet light absorption spectrum at a wavelength of 310 to 315 nm, and an absorption spectrum of visible light at 360 nm. The presence of an oxazolinium ion was determined by an absorption spectrum of ultraviolet light at 310 nm and by peaks of 2.37, 2.47, 4.00, 4.43 and 7.45 ppm in an NMR spectrum.

## Production Example 1

One mole of each of the olefins indicated in Table 1 and 300 ml of benzene were put in a vessel equipped with a stirrer, an internal heating device, a vapor condenser and a liquid-solid feed inlet, and with stirring, they were heated to 60 °C.

Each one mole of reagent (1) and reagent (2 were added, and the mixture was reacted for about 1 hour The reaction solvent and the unreacted materials were removed by distillation at 90 ° and 300 mmHg. Thus, additives A to C were obtained.

Table 1

| Additive | A | B | C |
|---|---|---|---|
| Compound having an unsaturated bond | 2-methyl-1-undecene | 1-eicosene | 7-tetradecene |
| Reagent (1) | benzylidenebutylamine and acetyl chloride | hydroxylbenzamide | beta-bromohydroximinoethylbenzene |
| Reagent (2) | tin tetrachloride | $BF_3$-ether complex | anhydrous sodium carbonate |
| Hetero ring | $>C=\overset{\oplus}{N}<$ | 1,3-oxazine structure | 1,3-oxazine structure |

## Production Example 2

One hundred grams of each of the oligomers indicated in Table 2 was dissolved in 500 ml of cyclohexane. The solution was put in a vessel equipped with a stirrer, an internal heating device, a vapor condenser and a liquid-solid feed inlet. With stirring, it was heated to 60 °C.

One mole of each of reagent (1) and reagent (2) were added, and the reaction was carried out for about 1 hour. Furthermore, 1.2 moles of reagent (3) was added and reacted.

A small amount of methanol was added to stop the reaction, and the reaction mixture was pouired into 1 liter of acetone/methanol (50/50) to coagulate the product. The product was dried by a vacuum dryer.

Thus, additives D to F of the invention were obtained.

Table 2

| Additive | D | E | F |
|---|---|---|---|
| Compound having a unsaturated bond | liquid isoprene (Mw = 4000) | styrene/butadiene block copolymer (Mw = 70000) | ethylene/propylene/diene copolymer (Mw = 100000) |
| Reagent (1) | benzilidene stearylamine benzoyl chloride | N-hydroxymethylthiobenzamide | alpha-chlorohydroxyimino propanol |
| Reagent (2) | titanium tetrachloride | tin tetrachloride | triethylamine |
| Reagent (3) | - | - | benzyl chloride |
| Hetero ring | $>C=\overset{\oplus}{N}<$ | 1,3-oxazine structure | $>C=\overset{\oplus}{N}<$ |

## Example 1

The effect of additives A, B, D and F obtained in Production Examples 1 and 2 to inhibit flocculation of inorganic fine particles dispersed in toluene was examined.

In each run, the dispersing effect of the additive was evaluated by the ratio of the average particle diameter (median diameter) of the additive dispersed by ultrasonication to that of the additive measured 10 minutes after the ultrasonication was stopped. The average particle size (micrometers) was measured by using a laser diffraction-type particle size meter (SK LASER MICRON SIZER PRO-100, supplied by Seishin Co., Ltd.). The measuring conditions of the average particle diameter were as follows:-

Amount of the inorganic fine particles: 50 -100 mg

Amount of toluene: 300 ml

The ratio of the average particle diameter 10 minutes after stopping ultrasonication, $[D_{50}]_0$ to the average particle of the inorganic fine particles 0 minute after stopping ultrasonication $[D_{50}]_0$, $[D_{50}]_{10}/[[D_{50}]_0$ was defined as the degree of flocculation. $D_{50}$ is the particle diameter for 50 % of the cumulative distribution of the particle diameters.

The results on various inorganic fine particles are shown in Tables 3 and 4. The amounts of the additives used are expressed as % by weight based on the inorganic fine particles.

Table 3

| Run No. | Inorganic fine particles | Degree of floccula-tion ( m) | none | A | B |
|---|---|---|---|---|---|
| | | | 0 | 5% | 5% |
| 1 | carbon black (HAF) (*1) | $[D_{50}]_0$<br>$[D_{50}]_{10}$<br>$\frac{[D_{50}]_{10}}{[D_{50}]_0}$ | 1.1<br>9.5<br>8.64 | 1.1<br>1.0<br>0.91 | 1.1<br>4.5<br>4.09 |
| 2 | zinc oxide (*2) | $[D_{50}]_0$<br>$[D_{50}]_{10}$<br>$\frac{[D_{50}]_{10}}{[D_{50}]_0}$ | 2.0<br>8.5<br>4.25 | 2.0<br>2.3<br>1.15 | 2.0<br>5.3<br>2.65 |
| 3 | alumina (*3) | $[D_{50}]_0$<br>$[D_{50}]_{10}$<br>$\frac{[D_{50}]_{10}}{[D_{50}]_0}$ | 5.0<br>12.5<br>2.50 | 5.0<br>5.1<br>1.02 | 5.0<br>8.1<br>1.62 |
| 4 | calcium carbonate (*4) | $[D_{50}]_0$<br>$[D_{50}]_{10}$<br>$\frac{[D_{50}]_{10}}{[D_{50}]_0}$ | 7.8<br>23.0<br>2.95 | 7.8<br>7.6<br>0.97 | 7.8<br>16.5<br>2.12 |

(*1): Asahi #70, a product of Asahi Carbon Co., Ltd.

(*2): Active zinc chemical, a product of Seido Chemical Co., Ltd.

(*3): "Higirite A-34", a product of Japan Light Metal Co., Ltd.

(*4): NS #100, a product of Nitto Powderization Co., Ltd.

Table 4

| Run No. | Inorganic fine particles | Degree of fluocculation (μm) | none | D | | | E | | |
|---|---|---|---|---|---|---|---|---|---|
| | | | 0% | 0.1% | 1.0% | 5.0% | 0.1% | 1.0% | 5.0% |
| 5 | Ferrite powder (*5) | $[D_{50}]_0$ | 2.0 | 2.0 | 2.0 | 2.0 | 2.0 | 2.0 | 2.0 |
| | | $[D_{50}]_{10}$ | 16.0 | 8.2 | 3.5 | 1.8 | 11.0 | 8.5 | 2.5 |
| | | $[D_{50}]_{10}/[D_{50}]_0$ | 8.0 | 4.10 | 1.75 | 0.90 | 5.50 | 4.25 | 1.25 |
| 6 | Cupric oxide (*6) | $[D_{50}]_0$ | 1.2 | 1.2 | 1.2 | 1.2 | 1.2 | 1.2 | 1.2 |
| | | $[D_{50}]_{10}$ | 7.3 | 7.0 | 5.6 | 1.8 | 7.3 | 6.4 | 5.2 |
| | | $[D_{50}]_{10}/[D_{50}]_0$ | 6.08 | 5.83 | 4.67 | 1.50 | 6.08 | 5.33 | 4.33 |
| 7 | Glass powder (*7) | $[D_{50}]_0$ | 10.3 | 10.3 | 10.3 | 10.3 | 10.3 | 10.3 | 10.3 |
| | | $[D_{50}]_{10}$ | 12.5 | 12.3 | 11.5 | 10.3 | 12.3 | 12.0 | 10.9 |
| | | $[D_{50}]_{10}/[D_{50}]_0$ | 1.21 | 1.19 | 1.12 | 1.00 | 1.19 | 1.17 | 1.06 |

(*5): Average particle diameter obtained by an electron microscope.

(*6): A product of Nisshin Chemical Co., Ltd.

(*7): PFA001, a product of Nitto Boseki Co., Ltd.

The results given in Tables 3 and 4 clearly show that the additives of the invention prevent flocculation of inorganic fine particles and are effective for dispersing them stably.

By increasing the amounts of the additives, their effects further increase.

## Example 2

Any of the additives A to F of the invention was added to a naphthenic process oil Diana Process Oil NM-280 (a product of Idemitsu Industries, Co., Ltd.) or toluene, and its effect on the viscosity was examined. The inorganic particles used were calcium carbonate NS#100, a product of Nitto Powderization

Co., Ltd.), clay (NN Kaolin Clay produced by Tsuchiya Kaolin Kogyo Co., Ltd.), silica (Hi-Sil), (a product of Pittsburgh Pateglass Co.), HAF carbon black (Asahi # 70, a product of Asahi Carbon Black Co.), talc (HITRON, a product of Takehara Chemical Industry Co.,), and titanium dioxide (Tipaque R-650, a product of Ishihara Sangyo kaisha, Ltd.). The results are shown in Table 5.

Table 5

| Run No. | 8 | 9 | 10 | 11 | 12 | 13 | 14 | 15 |
|---|---|---|---|---|---|---|---|---|
| Calcium carbonate (parts) | 35 | 50 | | | | | | |
| Carbon black (parts) | | | 15 | 25 | 35(*8) | | | |
| Silica (parts) | | | | | | 10 | 20 | |
| Clay (parts) | | | | | | | | 30 |
| Talc (parts) | | | | | | | | |
| Zinc oxide (parts) | | | | | | | | |
| Titanium oxide (parts) | | | | | | | | |
| Naphthenic oil (parts) | 65 | 50 | 85 | 75 | 65(*9) | 90 | 80 | 70 |
| Toluene (parts) | | | | | | | | |
| Additive (parts) A | 0.5 | 0.5 | | | | | | 1.0 |
| B | | | | | 1.5 | | | |
| C | | | | | | 3.0 | 3.0 | |
| D | | | 7.0 | 7.0 | | | | |
| F | | | - | | | | | |
| B-type viscosity (25°C, cps) | 430 (2300) | 5600 (35500) | 1100 (1900) | 8300 (22500) | 31000 (95000) | 120 (530) | 7400 (11600) | 330 (32000) |

(continued)

Note: The parenthesized figures are B-type viscosity measured in the absence of the additive.

(*8): FEF carbon black (Seast SO), a product of Tokai Carbon Co., Ltd.,

(*9): Flex M, a product of Fuji Kosan Co., Ltd.

Table 5 (continued)

| Run No. | 16 | 17 | 18 | 19 | 20 | 21 | 22 |
|---|---|---|---|---|---|---|---|
| Calcium carbonate (parts) | | | | | | | |
| Carbon black (parts) | | | | | | | |
| Silica (parts) | | | | | | | |
| Clay (parts) | 50 | | | | | | |
| Talc (parts) | | 20 | 40 | | | | |
| Zinc oxide (parts) | | | | 50 | 50 | | |
| Titanium oxide (parts) | | | | | | 30 | 50 |
| Naphthenic oil (parts) | 50 | 80 | 60 | 50(*9) | | 70(*9) | |
| Toluene (parts) | | | | | 50 | | 50 |
| Additive (parts) A | 1.0 | | | | | | 1.5 |
| B | | | | | | | |
| C | | | | | | 2.5 | |
| D | | | | | 2.5 | | |
| F | | 5.0 | 5.0 | 2.5 | | | |
| B-type viscosity (25°C, cps) | 2300 (98000) | 1100 (2800) | 4700 (12000) | 43000 (85000) | 33000 (98000) | 19000 (67000) | 2600 (10000) |

It is seen from Table 5 that by using the additives of this invention, dispersions having a substantially reduced B-type viscosity can be obtained. The marked decrease in viscosity can be noted in the case of calcium carbonate, carbon black, clay, titanium oxide and talc.

The decrease of viscosity in the dispersions makes it easy to mix the inorganic fillers with organic substances, and reduce the amount of energy required during mixing.

## Production Example 3

One mole of each of the alpha-olefins or polybutene indicated in Table 6 and 200 ml of benzene were put in a vessel equipped with a stirrer, an internally heating device, a vapor condenser, and a liquid-solid feed inlet.

One mole each of reagent (1) and reagent (2) shown in Table 6 were added, and the reaction was carried out for about 1 hour. Methanol was added to stop the reaction. Furthermore, 500 to 1 liter of methanol was added. The reaction mixture was then cooled in a refrigerator. The precipitate obtained was washed with methanol and dried. Thus, additives G to J were obtained.

Table 6

| Additive | G | H | I | J |
|---|---|---|---|---|
| Compound having an unsaturated bond at the ends | α-olefin (*10) $CH_3(CH_2)_{14}CH=CH_2$ | α-olefin (*11) $CH_3(CH_2)_{20}CH=CH_2$ | polybutene (Mw1000) | polybutene (Mw300) |
| Reagent (1) | benzylidene butylamine<br>acetyl chloride | benzylidene stearylamine<br>benzoyl chloride | benzylidene octylamine<br>acetyl chloride | benzylidene methylamine<br>propionyl chloride |
| Reagent (2) | tin tetrachloride | antimony pentachloride | titanium tetrachloride | tin chloride |

(*10) DIALENE 168; a $C_{13}$ - $C_{18}$ mixture, a product of Mitsubishi Kasei Corp.
(*11) DIALENE 208; a $C_{20}$ - $C_{28}$ mixture, a product of Mitsubishi Kasei Corp.

## Production Example 4

One hundred grams of each of the oligomers or the low-molecular weight polymers shown in Table 7 was dissolved in 500 ml of benzene or dichloromethane. The solution was put in a vessel equipped with a stirrer, an internal heating device, a vapor condenser and a liquid-solid feed inlet. With stirring, the solution was heated to 60 °C.

One mole each of the reagent (1) and reagent (2) were added and the mixture was reacted for about 1 hour. Furthermore, 2.0 moles of the reagent (3) was added and reacted.

After the reaction, the reaction mixture was poured into 1 liter of methanol to coagulate the product completely. The resulting liquid product was dried in a vacuum dryer.

Thus, additives K to N were obtained.

Table 7

| Additive | K | L |
|---|---|---|
| Compound having an unsaturated bond at the ends | α-olefin oligomer (*12)<br>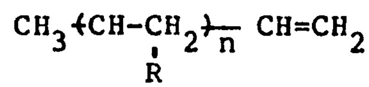 | polystyrylethyl methacrylate (*13)<br>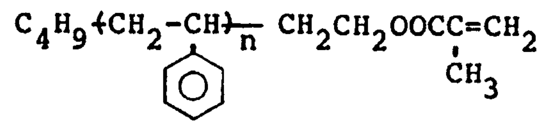 |
| Reagent (1) | 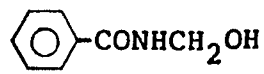 | 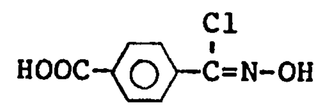 |
| Reagent (2) | Boron trifluoride etherate | Triethylamine |
| Reagent (3) | Benzyl chloride | Chloromethyl ether |

(continued)

(*12): Lipolube 400, molecular weight 800, a product of Lion K.K.

(*13): CHEMLINK 4500B, molecular weight 13,000, a product of Somar Corp.

Table 7 (continued)

| Additive | M | N |
|---|---|---|
| Compound having an unsaturated bond at the ends | polyethylene glycol dimethacrylate (*14) 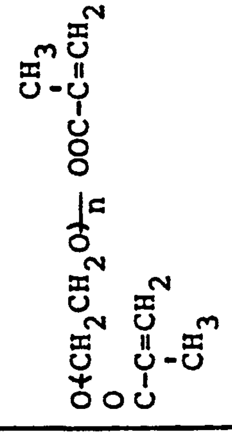 | polyisobutylene (*15) 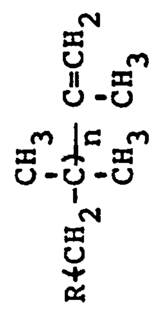 |
| Reagent (1) | 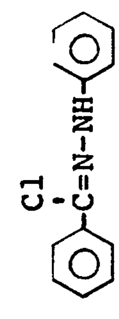 | 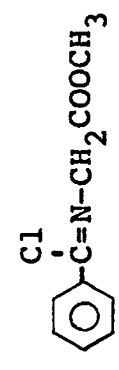 |
| Reagent (2) | Pyridine | Triethylamine |
| Reagent (3) | Dimethyl sulfate | Dimethyl sulfate |

(*14): NK ester-23G, n=23, a product of Shin-Nakamura Chemical K.K.

(*15): Tetrax-3T, molecular weight 30,000, a product of Nisseki Chemical Co., Ltd.

## Example 3

In the same way as in Example 1, the effect of the additives G, I and J to inhibit flocculation of inorganic fine particles dispersed in toluene was examined.

The results are shown in Table 8. The amounts of the additives are shown by % by weight based on the inorganic substance.

The results given in Table 8 clearly show that the additives of this invention prevent flocculation of the inorganic fine particles and stabilize their dispersion. It is also seen that by increasing the amount of the

additives, this effect is correspondingly increases.

Table 8

| Run No. | Inorganic fine particles | Degree of flocculation (μm) | Additive | | | | | | |
|---|---|---|---|---|---|---|---|---|---|
| | | | G | | I | | | J | |
| | | | 0% | 10% | 0% | 10% | 20% | 10% | 20% |
| 23 | Carbon black (HAF) | $[D_{50}]_0$<br>$[D_{50}]_{10}$<br>$\frac{[D_{50}]_{10}}{[D_{50}]_0}$ | 1.1<br>13<br>11.8 | 1.1<br>0.9<br>0.82 | | | | | |
| 24 | Titanium dioxide | $[D_{50}]_0$<br>$[D_{50}]_{10}$<br>$\frac{[D_{50}]_{10}}{[D_{50}]_0}$ | 2.1<br>35<br>16.7 | 2.2<br>2.3<br>1.05 | | | | | |
| 25 | Magnetic powder (*16) | $[D_{50}]_0$<br>$[D_{50}]_{10}$<br>$\frac{[D_{50}]_{10}}{[D_{50}]_0}$ | | | 1.8<br>21<br>11.7 | 1.8<br>2.3<br>1.28 | 1.8<br>1.8<br>1.00 | 1.9<br>2.0<br>1.05 | 1.9<br>1.9<br>1.00 |

(*16): BL-200, a product of Titan Kogyo K.K.

Example 4

The effect of the additive K or L on the viscosity of a naphthanic process oil (Diana Process Oil NM-280, a product of Idemitsu Industries, Co., Ltd.) in which titanium dioxide (Tipaque R-650, a product of Ishihara Sangyo Kaisha, Ltd.) or carbon black (Seast SO, FEF, a product of Tokai Carbon Co., Ltd. The results are shown in Table 9.

Table 9

| Run No. | 26 | 27 |
|---|---|---|
| Titanium dioxide (parts) | 40 | |
| Carbon Black (parts) | | 35 |
| Naphthenic oil (parts) | 60 | 65 |
| Additive (parts) K | 2 | |
| L | | 1.5 |
| B-type viscosity | 96400 | 31000 |
| (25 °C, cps) | (134000) | (95000) |

The use of the additives of this invention markedly reduced the viscosity of a dispersion of titanium dioxide or carbon black in the naphthenic process oil. Thus, the mere addition of the additives of the invention gives an effect of inhibiting the rising of the viscosity without requiring the addition of water or heating.

Example 5

Aluminum hydroxide (Higirite A-34, a product of Japan Light Metals , Co., Ltd) was dispersed in polyisoprene rubber [cis-1,4 = 98%, Nipol IR-2200, a product of Nippon Zeon Co., Ltd.] by using a small-sized kneader (Bravender Plastograph). The effect of the additive H on the viscosity of the dispersion was examined. The results are shown in Table 10.

Table 10

| Run No. | 28* | 29 |
|---|---|---|
| Aluminum hydroxide (parts) | 100 | 100 |
| Polyisoprene rubber (parts) | 100 | 100 |
| Additive H (parts) | - | 10 |
| Compound Mooney viscosity ($ML_{1+4}$, 100°C) | 63 | 51 |

(*) Comparison

The results show that the addition of the additives resulted in the decrease of the Mooney viscosity of the compound by 19 %.

## Example 6

Talc (Hitron, a product of Takehara Chemical Industry Co. Ltd.) or silica (Hi-sil 233, a product of Pittsburgh Plate Glass Co.) was dispersed in ethylene/propylene copolym (Esprene, a product of Sumitomo Chemical Co., Ltd.) by using a small-sized kneader (Bravender Plastograph). The effect of the additives M and N on the viscosity of the compound was examined. The results are shown in Table 11.

Table 11

| Run No. | 30 (*) | 31 | 32(*) | 33 |
|---|---|---|---|---|
| Ethylene/propylene copolymer Rubber (parts) | 100 | 100 | 100 | 100 |
| Talc (parts) | 100 | 100 | | |
| Silica (parts) | | | 75 | 75 |
| Additive (parts) M | 0 | 10 | | |
| N | | | 0 | 15 |
| Compound Mooney viscosity ($ML_{1+4}$, 100°C) | 95 | 81 | 124 | 101 |

(*) Comparative Example.

The effect of the additives of the invention to inhibit the rise of the viscosity can be clearly noted from the results shown in Tablees 10 and 11.

## Production Example 5

By the same procedure as in the production of the additive D except that a hydrogenation product of a (styrene/butadiene = 22/78, weight average molecular weight 70,000, iodine value 20) was used instead of the liquid polyisoprene. Thus, additive O was produced.

## Example 7

20 to 300 mg of organic fine particles (fine particles of nylon 12 having an average particle diameter of 7.4 micrometers or fine partricles of polyethylene terephthalate (PET) having an average particle diameter of 11.5 microns) was dispersed in 300 ml of toluene using the additive F or O. The effect of the additive to inhiibit floccuclation of the organic fine particles was evaluated as in Example 1. The amount of the additive used was 5 % based on the organic fine particles. The results are shown in Table 12.

Table 12

| Run No. | Organic fine particles | Degree of flocculation | Additive | | | |
|---|---|---|---|---|---|---|
| | | | F | | O | |
| | | | 0% | 5% | 0% | 5% |
| 34 | Nylon 12 | $[D_{50}]_0$<br>$[D_{50}]_{10}$<br>$\frac{[D_{50}]_{10}}{[D_{50}]_0}$ | 7.4<br>34.3<br>4.6 | 7.3<br>8.1<br>1.2 | | |
| 35 | PET | $[D_{50}]_0$<br>$[D_{50}]_{10}$<br>$\frac{[D_{50}]_{10}}{[D_{50}]_0}$ | | | 11.3<br>30.4<br>2.7 | 11.7<br>12.1<br>1.0 |

Production Example 6

Acetonitrile (6 ml), 1.98 g (20 mmol) of 2-ethyl-2-oxazoline and 0.186 g (1 mmol) of methyl p-toluenesulfonate were charged in a 230 ml glass vessel under a dry nitrogen. The temperature was returned to room temperature, and the solvent was evaporated. The residue was extracted with chloroform, and the extract was concentrated. It was then re-precipirated by using 150 ml of n-hexane to give 2.01 g (yield 97 %) of a white powdery polymer (additive P).

After the same polymerization as above was carried out, 0.54 g of water and 1.7 g of potassium carbonate as 24 hours. Then, the solvent was removed by evaporation. The residue was extracted from chloroform. The extract was concentrated and again precipitated with 150 ml of n-hexane to give 0.81 g of a white powdery polymer (additive P′).

Production Example 7

A polyolefin containing hydroxyl groups at both ends (Epol, a product of Idemitsu Petrochemical Co., Ltd.) was coagulated and purified by using benzene-methanol and then a portion (0.25 mol) of it was put into a 1-liter three-necked flask, and dissolved in 500 ml of benzene. 0.14 mole of p-toluenesulfonyl chloride was added, and at 15 °C or below, 80 ml of a 5N aqueous solution of sodium hydroxide was added dropwise. Subsequently, 0.14 mole of p-toluenesulfonyl cloride and 50 ml of a 5N aqueous solution of sodium hydroxide were added. The organic layer (benzene layer) was separated, washed with a 10% aqueous solution of sodium hydroxide, and dried over anhydrous sodium carbonate. The solvent was removed to give a polyolefin containing a tosylate group at both ends (yield 89 %).

17 mmoles of the tosylate-terminated polyolefin, 38 mmoles of 2-methyl-2-oxazoline and 50 ml of benzene were put in a 100 ml flask, and polymerized at 60 °C for 13 hours. The temperature returned to room temperature, and then, the contents of the flask were poured into 200 ml of methanol and coagulated. The yield of the polymer obtained (additive Q) was 96 %.

## Production Example 8

Polyepichlorohydrin (Gechron 1000, a product of Nippon Zeon Co., Ltd.) was coagulated and purified with benzene-methanol. After sufficient drying, this polymer was used in a reaction of introducing an oxazolinium ion.

A 100 ml glass vessel under a dry nitrogen atmosphere was charged with 50 ml of benzene, 10 mmol of 2-methyl-2-oxazoline and 7 mmols of the purified polyepichlorohydrin, and they were reacted at 80 ° for 8 hours. The temperature was returned to room temperature, and the reaction mixture was poured into 200 ml of methanol (additive R).

## Example 8

The effect of the additives of this invention P to R and the starting materials (comparative additives P′ to R′) to inhibit flocculation of various inorganic fine particles dispersed in methyl ethyl ketone was examined. The amount of the inorganic fine particles was 50 to 100 mg, and the amount of methyl ethyl ketone was 300 ml. The results are shown in Table 13.

The inorganic fine particles used were carbon black (MA 100, a product of Mitsubishi Kasei Corp.) titanium dioxide (Tipaque R-660) and a glass powder (PKFA-001, a product of Nitto Boseki Co., Ltd). The amounts of the additives were 6 or 10 % based on the inorganic parrticles.

The results given in Table 13 clearly showed that the additives of this invention prevent flocculation of the inorganicc fine particles and have an effect of stabilizing their dispersion.

### Table 13

| Run No. | 36 | 37(*) | 38 | 39(*) | 40 | 41(*) |
|---|---|---|---|---|---|---|
| Inorganic fine particles | Carbon black | | Titanium dioxide | | Glass powder | |
| Additive | P | P' | Q | Q' | R | R' |
| Amount(%) | 5% | 5% | 5% | 5% | 10% | 10% |
| $[D_{50}]_0$ (μm)<br>$[D_{50}]_{10}$ (μm) | 1.1<br>0.8 | 1.1<br>9.8 | 1.7<br>1.9 | 2.0<br>34.0 | 11<br>11 | 11<br>23 |
| $\frac{[D_{50}]_0}{[D_{50}]_{10}}$ | 0.7 | 8.9 | 1.1 | 17.0 | 1.0 | 2.1 |

(*) Comparison

## Example 9

The effect of the additives of the invention to inhibit flocculation of various organic fine particles dispersed in toluene was evaluated as in Example 1. The amount of the organic fine particles was 100 to 150 mg, and the amount of toluene was 300 ml.

The results are shown in Table 14.

The organic fine particles used were benzoguanamine fine particles (Epostar, a producct of Japan Catalytic Chemical Co., Ltd.), and methacrylate resin fine particles (MP, a product of Soken Chemical Co., Ltd.). The amounts of the additives used were 3 % based on the organic fine particles.

The results given n Table 14 clearly show that the additives of this invention prevent flocculation of the organic fine powders, and have an effect of stabilizing their dispersion.

Table 14

| Run No. | 42 | 43(*) | 44 | 45(*) |
|---|---|---|---|---|
| Organic fine particles | benzoguanamine resin particles | | methacrylate particles | |
| Additive | Q | Q' | R | R' |
| $[D_{50}]_0$ (μm) | 1.1 | 1.2 | 13.5 | 13.6 |
| $[D_{50}]_{10}$ (μm) | 1.3 | 12.1 | 14.1 | 94.5 |
| $\frac{[D_{50}]_{10}}{[D_{50}]_0}$ | 1.2 | 10.1 | 1.0 | 6.9 |

(*) Comparison

## Production Example 9

A 2-liter stainless steel polymerization vessel was washed, dried and purged with dry nitrogen and 50 g of 1,3-butadiene, 100 g of styrene, 820 g of benzene, 0.5 millimole of diethylene glycol dimethyl ether and the amount indicated in Table 15 of an n-hexane solution of n-butyl lithium were added. With stirring, the monomers were polymerized at 60 °C for 2 hours. After the polymerization, each of the modifiers indicated in Table 15 was added, and the mixture was stirred for 30 minutes. The polymer solution in the polymerizartion reactor was transferred to a 2.0 % methanol solution of 2,6-di-t-butyl-p-cresol to coagulate the resulting polymer. It was then dried at 60°C under reduced pressure for 24 hours.

As a result, additives S to V and V′ were obtained.

The amount of styrene was determined by an infrared aspectrophotometer [Hampton, Anal. Chem., 21, 923 (1949)]. The weight average molecular weights of the addivives were measured by gel permeation chromatography under the following conditions (calculated for standard polystyrene).

Table 15

| Additive | n-butyl lithium (millimole) | Modifier | | Structure | |
|---|---|---|---|---|---|
| | | Compound | millimole | Amount of styrene (%) | Weight average molecular weight |
| S | 1.3 | 4,4′-bis(diethylamino) benzophenone | 1.5 | 66 | $155 \times 10^3$ |
| T | 30 | ditto | 35 | 68 | $5 \times 10^3$ |
| U | 0.5 | ditto | 0.8 | 66 | $810 \times 10^3$ |
| V | 1.3 | N-methyl-2-pyrrolidone | 1.5 | 67 | $160 \times 10^3$ |
| V′ | 1.3 | — | - | 68 | $170 \times 10^3$ |

## Example 10

The effect of the additives S-B and V′ (comparison) on the dispersion of particulate substances in toluene was evaluated as in Example 1. The results are shown in Table 16.

The inorganic fine particles used were the same carbon black, zinc oxide, alumina and calcium carbonate as used in Example 1. The organic fine particles were the nylon 12 and PET used in Example 8.

| Measuring conditions | |
|---|---|
| Inorganic fine particles | 50 to 100 mg |
| Toluene | 350 ml |
| Organic fine particles | 20 to 300 mg |
| Toluene | 300 ml |

The amount of the additive used is expressed by % by weight based on the particulate substance.

Table 16

| Run No. | Fine particles | Degree of flocculation (μm) | Additive | | | | | | |
|---|---|---|---|---|---|---|---|---|---|
| | | | None | S | | | T | | |
| | | | 0% | 0.1% | 1.0% | 5.0% | 0.1% | 1.0% | 5.0% |
| 46 | Carbon black | $[D_{50}]_0$<br>$[D_{50}]_{10}$<br>$\frac{[D_{50}]_{10}}{[D_{50}]_0}$ | 1.1<br>9.5<br>8.64 | 1.1<br>1.2<br>1.09 | 1.1<br>1.1<br>1.00 | 1.1<br>1.1<br>1.00 | 1.1<br>1.3<br>1.18 | 1.1<br>1.1<br>1.00 | 1.1<br>1.1<br>1.00 |
| 47 | Zinc oxide | $[D_{50}]_0$<br>$[D_{50}]_{10}$<br>$\frac{[D_{50}]_{10}}{[D_{50}]_0}$ | 2.0<br>8.5<br>4.25 | 2.0<br>2.9<br>1.45 | 2.0<br>2.7<br>1.35 | 2.0<br>2.5<br>1.25 | 2.0<br>2.7<br>1.35 | 2.0<br>2.4<br>1.20 | 2.0<br>2.3<br>1.15 |
| 48 | Alumina | $[D_{50}]_0$<br>$[D_{50}]_{10}$<br>$\frac{[D_{50}]_{10}}{[D_{50}]_0}$ | 5.0<br>12.5<br>2.50 | 5.0<br>7.9<br>1.96 | 5.0<br>6.9<br>1.38 | 5.0<br>5.5<br>1.10 | 5.0<br>7.5<br>1.50 | 5.0<br>6.1<br>1.22 | 5.0<br>5.1<br>1.05 |

(continued)

Table 16 (continued)

| Run No. | Fine particles | Degree of flocculation (μm) | Additive | | | | | | |
|---|---|---|---|---|---|---|---|---|---|
| | | | None | S | | | T | | |
| | | | 0% | 0.1% | 1.0% | 5.0% | 0.1% | 1.0% | 5.0% |
| 49 | Calcium carbonate | $[D_{50}]_0$<br>$[D_{50}]_{10}$<br>$\frac{[D_{50}]_{10}}{[D_{50}]_0}$ | 7.8<br>23.0<br>2.95 | 7.8<br>11.0<br>1.41 | 7.8<br>9.3<br>1.19 | 7.8<br>8.1<br>1.04 | 7.8<br>10.3<br>1.32 | 7.8<br>8.5<br>1.09 | 7.8<br>7.7<br>0.90 |
| 50 | Nylon 12 | $[D_{50}]_0$<br>$[D_{50}]_{10}$<br>$\frac{[D_{50}]_{10}}{[D_{50}]_0}$ | 7.4<br>34.3<br>4.64 | | | 7.6<br>8.8<br>1.16 | | | |
| 51 | PET | $[D_{50}]_0$<br>$[D_{50}]_{10}$<br>$\frac{[D_{50}]_{10}}{[D_{50}]_0}$ | 11.5<br>30.7<br>2.67 | | | | | | |

(continued)

Table 16 (continued)

| Run No. | Fine particles | Degree of flocculation (μm) | Additive | | | | | | | | |
|---|---|---|---|---|---|---|---|---|---|---|---|
| | | | U | | | V | | | V' | | |
| | | | 0.1% | 1.0% | 5.0% | 0.1% | 1.0% | 5.0% | 0.1% | 1.0% | 5.0% |
| 46 | Carbon black | $[D_{50}]_0$<br>$[D_{50}]_{10}$<br>$\frac{[D_{50}]_{10}}{[D_{50}]_0}$ | 1.1<br>4.5<br>4.09 | 1.1<br>3.0<br>2.73 | 1.1<br>2.8<br>2.55 | 1.1<br>1.2<br>1.09 | 1.1<br>1.1<br>1.00 | 1.1<br>1.1<br>1.00 | 1.1<br>9.5<br>8.64 | 1.1<br>9.3<br>8.45 | 1.1<br>9.2<br>8.36 |
| 47 | Zinc oxide | $[D_{50}]_0$<br>$[D_{50}]_{10}$<br>$\frac{[D_{50}]_{10}}{[D_{50}]_0}$ | 2.0<br>6.5<br>3.25 | 2.0<br>5.1<br>2.55 | 2.0<br>4.1<br>2.05 | 2.0<br>2.5<br>1.25 | 2.0<br>2.3<br>1.15 | 2.0<br>2.2<br>1.10 | 2.0<br>8.6<br>4.30 | 2.0<br>8.5<br>4.25 | 2.0<br>8.3<br>4.15 |
| 48 | Alumina | $[D_{50}]_0$<br>$[D_{50}]_{10}$<br>$\frac{[D_{50}]_{10}}{[D_{50}]_0}$ | 5.0<br>12.5<br>2.50 | 5.0<br>10.5<br>2.10 | 5.0<br>8.2<br>1.84 | 5.0<br>7.6<br>1.52 | 5.0<br>6.4<br>1.28 | 5.0<br>5.0<br>1.00 | 5.0<br>13.0<br>2.60 | 5.0<br>13.0<br>2.60 | 5.0<br>12.5<br>2.5 |

(continued)

Table 16 (continued)

| Run No. | Fine particles | Degree of flocculation ( m) | Additive | | | | | | | | |
|---|---|---|---|---|---|---|---|---|---|---|---|
| | | | U | | | V | | | V' | | |
| | | | 0.1% | 1.0% | 5.0% | 0.1% | 1.0% | 5.0% | 0.1% | 1.0% | 5.0% |
| 49 | Calcium carbonate | $[D_{50}]_0$<br>$[D_{50}]_{10}$<br>$\frac{[D_{50}]_{10}}{[D_{50}]_0}$ | 7.8<br>21.5<br>2.76 | 7.8<br>16.5<br>2.12 | 7.8<br>12.3<br>1.58 | 7.8<br>10.9<br>1.40 | 7.8<br>9.3<br>1.19 | 7.8<br>7.9<br>1.01 | 7.8<br>23.5<br>3.01 | 7.8<br>23.0<br>2.95 | 7.8<br>22.5<br>2.88 |
| 50 | Nylon 12 | $[D_{50}]_0$<br>$[D_{50}]_{10}$<br>$\frac{[D_{50}]_{10}}{[D_{50}]_0}$ | | | | | | | | | |
| 51 | PET | $[D_{50}]_0$<br>$[D_{50}]_{10}$<br>$\frac{[D_{50}]_{10}}{[D_{50}]_0}$ | | | | | | 11.9<br>12.4<br>1.04 | | | |

## Example II

The effect of the additive S of the invention on the incorporation of 40 % of calcium carbonate in polypropylene was examined.

To ascertain the effect of the method of mixing on the properties of the final composition, two methods were used.

A first testing method was to dry-blend calcium carbonate and a dispersant for 1 minute at 3600 rpm in a Henschel mixer in advance. In this method, the temperature was initially room temperature, but rose during the mixing operation. Calcium carbonate so surface-treated with the dispersant was dry-blended with polypropylene. The blend was molded at 230 °C by screw injection to prepare a test piece having a longitudinal size of 10 mm, a lateral size of 10 mm and a thickness of 1 mm.

A second testing method involved transferring the blend of the above three materials from the Henschel mixer to a high shear double concentric mixer, and mixed at 230 °C under high shear, and then injection mold the blend at 230 °C to prepare a test piece.

The results are shown in Table 17. The amount of the additive used is shown by % by weight based on polypropylene.

the results given in Table 17 show that polypropylene containing calcium carbonte treated with the additive of this invention has improved properties over the case of using the untreated material.

The addition of the additive of this invention in an amount of 1.0 % brought about a marked improvement in impact strength. By using the double concentric screw, further improved properties were obtained. It is presumed that by the mixing under a high shear, the reactivity between the additive and the inorganic material was improved.

Table 17

| Run No. | Compounding receipt | Tensile strength ($kg/cm^2$) | Flexural stress ($kg/mm^2$) | Izod impact strength (notched) (kg-cm/cm) |
|---|---|---|---|---|
| 52(*) | No calcium carbonate | 350 | 1.7 | 3.8 |
| 53(*) | Calcium carbonate | 450 | 6.7 | 2.2 |
| 54 | Calcium carbonate additive 0.5 % | 390 | 4.7 | 3.0 |
| 55 | Calcium carbonate additive 1.0 % | 350 | 4.1 | 5.5 |
| 56 | Calcium carbonate(**) | 320 | 3.3 | 10.0 |

(*) Comparison

(**) According to method 2.

## Production Example 10

One hundred grams of each of the polymers shown in Table 18 was dissolved in 500 ml of benzene, and the solution was put in a vessel equipped with a stirrer, an internally heating device, a vapor condenser and a liquid-solid feed inlet. With stirring, the solution was heated to 60 °C. The reagent (1) and the reagent (2) were added in an amount of 0.1 mole each and reacted for about 1 hour. (As required, 0.15 mole of the reagent (3) was added.) Then, a small amount of methanol was added to stop the reaction. The reaction mixture was poured into 1 liter of methanol to coagulate the product. The product was dried by a vacuum dryer.

Thus, additives W and X were obtained.

Table 18

| Additive | W | X |
|---|---|---|
| Compound having an unsaturated bond | Polybutene (Mw = 3,750) | ethylene/propylene/diene copolymer (Mw = 100,000) |
| Reagent (1) | benzylidene stearylamine and acetyl chloride) | N-hydroxymethyl benzamide |
| Reagent (2) | Tin tetrachloride | boron trifluorided etherate |
| Reagent (3) | - | p-toluenesulfonic acid |

## Production Example 11

A 2-liter stainless steel polymerization reactor was washed, dried, and purged with dry nitrogen, and 150 g of 1,3-butadiene, 50 g of styrene, 890 g of benzene, 0.5 millimole of tetramethylethylenediamine and 1.8 millimoles of an n-hexane solution of n-butyl lilthium were added. With stirring, the monomers were polymerized at 40 °C for 1 hour.

After the polymerization, 1.0 millimole of N-methylpyrrolidone was added and reacted with stirring for 10 minutes. Then, the contents in the reactor were poured into a 2.0 % methanol solution of 2,6-di-t-butyl-p-cresol to coagulate the resulting polymer. The polymer was then dried at 60 °C and reduced pressure for 24 hours. The resulting polymer was designated as additive Y.

A polymer was produced under the same conditions as above except that N-methylpyrrolidone was not reacted. The resulting polymer was designated as additive Y′.

The additives Y and Y′ had a bonded styrene content of 25 %, and a weight average molecular weight of 150,000 as measured by GPC (calculated for polystyrenes).

## Production Example 12

Ethylene/propylene/diene copolymer (Mw = 100,000) (160 g) and 3 liters of benzene were put into a vessel equiepped with a stirrer, an internal heating device, a vapor condenser and a liquid-solid feed inlet. Benzylidene butylamine (20 millimoles) and 20 millimoles of acetyl chloride were added each as a benzene solution. Furthermore, 0.5 to 1 liter of methanol was added. The reaction mixture was cooled in a refrigenerator. The resulting precipitate was washed with methanol, and dried to give an additive Z. The starting material was designated as additive Z′.

## Example 12

Additives O and W to Y and additive Y′ (comparison) were each added to each of the following fuel oils in an amount of 300 ppm or 500 ppm. The low-temperature flowability and the detergent-dispersibilitry of the oils were evaluated. The results are shown in Table 19.

Fuel oil A: 20 % boiling point 268 °C, 90 % boiling point 345 °C, dring point 373 °C

Fuel oil B: 20 % boiling point 221 °C, 90 % boiling point 332 °C, drying point 359 °C.

As measures of the low-temperature flowability of a petroleum intermediate distilled fuel oil, the pour point in accordance with JIS K-2204, and cold filter plugging filter (CFPP) in accordance with JSS K-2288 were measured. To determine detergent-dispersibility, an oxidation stability test of a lubricant was conducted in accordance with JIS K-2514, and by observing the state of adhesion of a lacquor to a vanish rod,

the detergent-dispersibility was evaluated.

The data given in Table 19 show that the additives to fuel oils in accordance with this invention have the function of a low-temperature flowability improver and a dispersant.

Table 19

| Run No. | Fuel oil | Additive | | Flowability | | Detergent-dispersibility |
|---|---|---|---|---|---|---|
| | | Type | amount (ppm) | pour point (°C) | CFPP (°C) | Degree of laquor adhesion |
| 57<br>58<br>59 | A | W<br>X<br>Y | 500<br>300<br>500 | -13.5<br>-17.5<br>-12.5 | -11.0<br>-14.0<br>- 9.0 | No adhering matter |
| 60(*)<br>61(*)<br>62(*) | | -<br>W'<br>Y' | -<br>500<br>500 | - 7.5<br>-10.0<br>- 9.5 | - 5.0<br>- 8.0<br>- 7.0 | No adhering matter |
| 63<br>64<br>65 | B | X<br>P<br>Y | 500<br>300<br>300 | -22.5<br>-15.5<br>-14.5 | -17.0<br>-13.0<br>-12.5 | No adhering matter<br>adhesion thin<br>ditto |
| 66(*)<br>67(*)<br>68(*) | | -<br>X'<br>Y' | -<br>300<br>300 | - 9.0<br>-11.5<br>-10.5 | - 6.5<br>- 8.5<br>- 8 | Adhesion thick<br>ditto<br>ditto |

(*) indicates a comparison

## Example 13

Each of the additives Y, Y', Z, Z', G to J and G' to J' (G' to J' are the starting materials for the production of additives G to J) was added in an amount of 5 parts to 95 parts of a hydrogenated purified paraffinic mineral oil. The viscosity index of the resulting lubricant oil was measured in accordance with JIS K-2283. As a measure of shearing stability, the viscosity loss rate (calculated from the following equation) by ultrasonic radiation was measured in accordance with JPI-5S-29-88.

$$\text{Viscosity loss rate (\%)} = \frac{V_o - V_f}{V_o} \times 10^6$$

$V_o$ is the dynamic viscosity before ultrasonic radiation (cst), and $V_f$ is the dynamic viscosity after the ultrasonic radiation (cst). The results are shown in Table 20.

The detergent-dispersibility was evaluated by a test accordance JIS K-2514 from the degree of adhesion of a lacquor to a varnish rod. The results are shown in Table 21.

Table 20

| Run No. | Additive | Dynamic viscosity (cst) | | | V.I. | Low viscosity reduction rate (%) |
|---|---|---|---|---|---|---|
| | | 40°C | 100°C | 100°C (**) | | |
| 69(*) | - | 28.50 | 5.00 | | 100 | |
| 70 | Y | 45.44 | 9.31 | 9.18 | 193 | 1.40 |
| 71 | G | 39.01 | 6.28 | 5.71 | 139 | 9.08 |
| 72 | H | 36.54 | 6.66 | 6.01 | 140 | 9.76 |
| 73 | I | 37.54 | 6.98 | 6.72 | 145 | 3.72 |
| 74 | J | 40.01 | 7.55 | 7.28 | 159 | 3.68 |
| 75 | Z | 44.83 | 9.71 | 9.54 | 209 | 1.75 |

(*) comparison
(**) After the ultrasonication

Table 21

| Run No. | | Detergent-dispersibility |
|---|---|---|
| | | Degree of lacquor adhesion |
| 76 | Y | No adhesion |
| 77 | G | Adhesion thin |
| 78 | H | No adhesion |
| 79 | I | Adhesion thin |
| 80 | J | No adhesion |
| 81 | Z | No adhesion |
| 82(*) | - | Adhesion thick |
| 83(*) | Y' | Adhesion thick |
| 84(*) | G' | Adhesion thick |
| 85(*) | H' | Adhesion thick |
| 86(*) | I' | Adhesion thick |
| 87(*) | J' | Adhesion thick |
| 88(*) | Z' | Adhesion thick |

(*) Comparison

## Claims

1. An additive for improving the dispersibility of a particulate substance in an organic medium or a thermoplastic polymer, said additive comprising at least one compound selected from the group consisting of compounds having a 1,3-oxazine structure in the molecule, compounds having a $>C=\overset{\oplus}{N}<$ bond in the molecule and compounds having an oxazolinium ion in the molecule.

2. The additive of claim 1 in which the particulate substance is an inorganic or organic substance.

3. The additive of claim 1 in which the organic medium is selected from organic solvents, lubricant oils and fuel oils.